(19) Europäisches Patentamt
European Patent Office
Office européen des brevets

(11) Veröffentlichungsnummer: **0 001 065**
**B1**

(12) # EUROPÄISCHE PATENTSCHRIFT

(21) Anmeldenummer: **78100710.9**

(22) Anmeldetag: **21.08.78**

(51) Int. Cl.³: **C 07 D 239/04**
C 07 D 233/02,
C 08 G 18/80

(54) **Cycloaminale, ein Verfahren zu ihrer Herstellung, sowie ihre Verwendung**

(30) Priorität: **01.09.77 DE 2739313**

(43) Veröffentlichungstag der Anmeldung:
**21.03.79 Patentblatt 79/06**

(45) Bekanntmachung des Hinweises auf die Patenterteilung:
**23.07.80 Patentblatt 80/15**

(84) Benannte Vertragsstaaten:
**BE DE FR GB NL SE**

(56) Entgegenhaltungen:
**DE - A - 2 156 881**

(73) Patentinhaber: **Bayer, AG**
**Zentralbereich Patente, Marken und Lizenzen**
**D - 5090 Leverkusen 1, Bayerwerk (DE)**

(72) Erfinder: **Hajek Manfred, Dr.**
**Hahnenweg 1**
**D - 5000 Köln 80 (DE)**
**Wagner, Kuno, Dr.**
**Am Kiesberg 8**
**D - 5090 Leverkusen 1 (DE)**
**Uerdingen, Walter, Dr.**
**Im Aehlemaar 17**
**D - 5060 Bergisch-Gladbach 2 (DE)**
**Wellner, Wolfgang, Dr.**
**Hahnenweg 8**
**D - 5000 Köln 80 (DE)**

**EP 0 001 065 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Cycloaminale, ein Verfahren zu ihrer Herstellung, sowie ihre Verwendung

Gegenstand der vorliegenden Erfindung sind Verbindungen der Formel

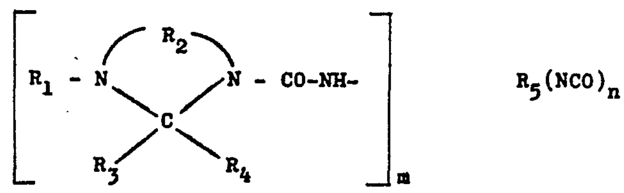

in welcher

m für eine ganze Zahl von 1 bis 3 und

n für 0, 1 oder 2 steht, wobei die Summe m + 2 oder 3 beträgt,

$R_1$ einen gegebenenfalls Cyano-substituierten aliphatischen Kohlenwasserstoffrest mit 1 bis 6 Kohlenstoffatomen, einen cycloaliphatischen Kohlenwasserstoffrest mit 5—10 Kohlenstoffatomen oder einen araliphatischen Kohlenwasserstoffrest mit 7—10 Kohlenstoffatomen bedeutet,

$R_2$ für einen zweiwertigen aliphatischen Kohlenwasserstoffrest mit 2 bis 6 Kohlenstoffatomen steht, wobei zwischen den beiden Stickstoffatomen mindestens zwei Kohlenstoffatome angeordnet sind,

$R_3$ und $R_4$ für gleiche oder verschiedene Reste stehen und Wasserstoff, aliphatische Kohlenwasserstoffreste mit 1—18 Kohlenstoffatomen, cycloaliphatische Kohlenwasserstoffreste mit 5—10 Kohlenstoffatomen oder aromatische Kohlenwasserstoffreste mit 6—10 Kohlenstoffatomen bedeuten oder wobei die beiden Reste $R_3$ und $R_4$ zusammen mit dem Kohlenstoffatomen des heterocyclischen Rings auch einen 5- oder 6-gliedrigen cycloaliphatischen Ring bilden können,

$R_5$ für einen (m + n)-wertigen Rest steht, wie er durch Entfernung von m + n Isocyanatgruppen aus einem (m + n)-wertigen Polyisocyanat erhalten wird.

Gegenstand der vorliegenden Erfindung ist auch ein Verfahren zur Herstellung dieser Verbindungen, welches dadurch gekennzeichnet ist, daß man cyclische Aminale der Formel

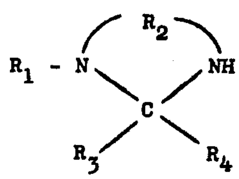

mit Polyisocyanaten der Formel

$$R_5(NCO)_{m+n}$$

umsetz, wobei

$R_1$, $R_2$, $R_3$, $R_4$, $R_5$, m und n die obengenannte Bedeutung haben.

Gegenstand der vorliegenden Erfindung ist auch die Verwendung der erstgenannten Formel entsprechenden Isocyanatgruppenfreien Verbindungen als durch Feuchtigkeit und/oder Hitze aktivierbare Vernetzer für Kunststoffvorläufer mit freien oder mit an sich bekannten Blockierungsmitteln für Isocyanatgruppen blockierten Isocyanatgruppen.

Gegenstand der vorliegenden Erfindung ist schließlich auch die Verwendung der erstgenannter Formel entsprechenden Verbindungen mit freien Isocyanatgruppen als unter dem Einfluß von Feuchtigkeit und/oder Hitze zu hochmolekularen Kunststoffen ausreagierende Kunststoffvorläufer.

In diesen Formeln und auch nachstehend haben $R_1$, $R_2$, $R_3$, $R_4$, $R_5$ m und n die obengenannte Bedeutung. Vorsugsweise stehen

$R_1$ für einen aliphatischen Kohlenwasserstoffrest mit 1—3 Kohlenstoffatomen, welcher im Falle des Vorliegens von mindestens 2 Kohlenstoffatomen gegebenenfalls in 2-Stellung einen Cyanosubstituenten aufweist, oder für einen Cyclohexylrest,

$R_2$ für einen Di- oder Trimethylenrest

$R_3$ und $R_4$ unabhängig voneinander für Wasserstoff, einen aliphatischen Kohlenwasserstoffrest

mit 1—3 Kohlenstoffatomen oder zusammen mit dem Kohlenstoffatom des heterocyclischen Rings einen Cyclohexylrest,

$R_5$ für einen zweiwertiger Rest, wie er durch Entfernung der Isocyanatgruppen aus Hexamethylendiisocyanat, 2,4- und/oder 2,6-Diisocyanatotoluol, 4,4'-Diisocyanatodiphenylmethan oder 1-Isocyanato-3,3,5-trimethyl-5-isocyanatomethylcyclohexan erhalten wird,

n für 0 oder 1 und

m für 1 oder 2, wobei die Summe n + m zwei beträgt.

Polyamine sind grundsätzlich interessante Reaktionspartner für das Isocyanatpolyadditionsverfahren, da sie Produkte ergeben, die die stabile Harnstoffverknüpfung enthalten. Nachteilig ist in diesem Zusammenhang die hohe Reaktivität dieser Verbindungsklasse, wodurch schon ein normales Mischen der Reaktionspartner erschwert und die Herstellung von Mischungen mit langer Topfzeit in oder Lagerzeit überhaupt verhindert werden. Bei vielen Aminen wird ihre Verwendung durch die Flüchtigkeit und die physiologische Wirkung dieser Verbindungen noch weiter eingeschränkt.

Diese Nachteile dieser technisch interessanten Substanzklasse können bei Verwendung der erfindungsgemäßen Cycloaminale vermieden werden.

Die erfindungsgemäßen Cycloaminale ergeben in Abwesenheit von Feuchtigkeit mit Polyisocyanaten lagerstabile Mischungen, was überraschend ist, da von cyclischen Aminalen wie z.B. N,N'-substituierten Imidazolidinen bekannt ist (vgl. H.Böhme, W.Pasche, Arch.Pharmaz. *302* (1969) 2, 81—90), daß sie mit Isocyanaten unter Einschubreaktionen zu Triazacycloheptanonderivaten reagieren. Die Cycloaminale selbst werden leicht durch Feuchtigkeit gespalten und liefern neben der Carbonylverbindung $R_3R_4—C=O$ eine gegen Isocyanat reaktive Verbindung mit den Endgruppen

$$R_1-\underset{H}{\underset{|}{N}}-R_2-NH-CO-NH-$$

Die Isocyanatgruppen-freie Cycloaminale stellen somit potentielle Reaktionspartner für organische Polyisocyanate dar, und Gemische derartiger Polycycloaminale mit Polyisocyanaten sind daher durch Wasser hartbare Systeme, wobei eine Cycloaminalgruppe jeweils nur eine sek. Aminofunktion liefert, da die 2. NH-Gruppe Teil einer Harnstoffgruppe und somit gegenüber Isocyanatgruppen sehr reaktionsträge ist. So entspricht die Funktionalität der erfindungsgemäßen blockierten Amine genau der Anzahl an Cycloaminaleinheitem im Molekül. Der Abstand zwischen den verkappten sek. Aminogruppen und die maximale Funktionalität können durch geeignete Auswahl des Isocyanatderivats bestimmt werden, sind dadurch in weiten Grenzen veränderlich und köhnen so den Erfordernissen der verschiedensten Anwendungsmöglichkeiten optimal angepaßt werden.

Zur Herstellung der erfindungsgemäßen Verbindungen nach dem erfindungsgemäßen Verfahren kann man von N-substituierten cyclischen Aminalen der allgemeinen Formel

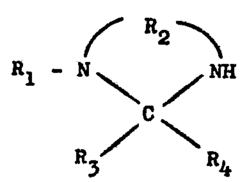

ausgehen und diese mit polyfunktionellen Isocyanaten der allgemeinen Formel

$$R_5(NCO)_{m+n}$$

umsetzen.

Die für das erfindungsgemäße Verfahren benötigten Cycloaminale sind durch cyclisierende Kondensation von monosubstituierten Diaminen der Formel

$$R_1-\underset{H}{\underset{|}{N}}-R_2-NH_2$$

mit Aldehyden oder Ketonen der allgemeinen Formel

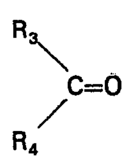

unter Wasserabspaltung nach literaturbekannten Methoden zugänglich.

Als N-monosubstituierte Diamine der allgemeinen Formel

$$R_1-\overset{\displaystyle H}{\overset{|}{N}}-R_2-NH_2$$

eignen sich bevorzugt Derivate des Äthylendiamins, 1,2-Propylen-, Trimethylen- und Tetramethylendiamin, wobei der Rest $R_1$ beispielsweise ein niederer Alkylrest ist, wie z.B. eine Methyl-, Äthyl-, Propyl-, i-Propyl-, n-Butyl-, i-Butyl- oder t-Butylgruppe. Weiter eignen sich die Monoadditionsprodukte der Diamine

$$H_2N-R-NH_2$$

an aktivierten Doppelbindungssysteme, z.B. Derivate der Acrylsäure, z.B. Acrylnitril oder Methacrylnitril.

Desweiteren eignen sich z.B. auch die N-Cyclohexyl-, N-Benzyl- und N-Phenylderivate der genannten Diamine.

Als Carbonylverbindungen eignen sich beispielsweise nachstehend angeführte Aldehyde und Ketone: Formaldehyd, Acetaldehyd, Isobutraldehyd, Benzaldehyd, Aceton, Methyläthylketon, Diäthylketon, Methylisobutylketon, Diisobutylketon, Cyclopentanon und Cyclohexanon.

Als Polyisocyanatkomponente $R_5\ (NCO)_{m+n}$ kommen sowohl aliphatische, als auch cycloaliphatische, araliphatische, aromatische oder heterocyclische Polyisocyanate in Betracht, wie sie z.B. von W.Siefgen in Justus Liebigs Annalen der Chemie, 562, Seiten 75 bis 136, beschrieben werden, beispielsweise Äthylen-diisocyanat, 1,6-Hexamethylendiisocyanat, 1,12-Doedecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanatomethyl-cyclohexan (DAS 1 202 785), 2,4- und 2,6-Hexahydrotoluylendiisocyanat sowie beliebige Gemische dieser Isomeren, Perhydro-2,4'- und/oder -4,4'-diphenylmethan-diisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4',4"-triisocyanat, Polyphenylpolymethylen-polyisocyanate, wie sie durch Anilin-Formaldehyd-Kondensation und anschließende Phosgenierung erhalten und z.B. in den britischen Patentschriften 874 430 und 848 671 beschrieben werden.

Carbodiimidgruppen aufweisende Polyisocyanate, wie sie in der deutschen Patentschrift 1 092 007 beschrieben werden, Allophanatgruppen aufweisende Polyisocyanate, wie sie z.B. in der britischen Patentschrift 994 890, Isocyanuratgruppen aufweisende Polyisocyanate, wie sie z.B. in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der amerikanischen Patentschrift 3 394 164 beschrieben werden, Biuretgruppen aufweisende Polyisocyanate, wie sie z.B. in der deutschen Patentschrift 1 101 394 beschrieben.

Es ist auch möglich, beliebige Mischungen der vorgenannten Polyisocyanate zu verwenden.

Die bevorzugten beim erfindungsgemäßen Verfahren einzusetzenden Polyisocyanate sind solche der Formel

$$R_5\ (NCO)_{m+n}$$

für welche

$R_5$, m und n die obengenannte bevorzugte Bedeutung haben.

Dies bedeutet, das die bevorzugt beim erfindungsgemäßen Verfahren einzusetzende Isocyanat-Komponente aus dem bevorzugten Diisocyanaten der Polyurethan-Chemie wie z.B. Hexamethylen-diisocyanat, 2,4- und/oder 2,6-Diisocyanatotoluol, 4,4'-Diisocyanatodiphenylmethan oder 1-Isocyanato-3,3,5-trimethyl-5-isocyanatomethyl-cyclohexan (IPDI) besteht. Außer derartigen einfachen Diisocyanaten können auch aus derartigen Diisocyanaten und unterschüssigen Mengen an Polyhydroxylverbindungen der in der Polyurethan-Chemie an sich bekannten Art hergestellte NCO-Präpolymere als Polyisocyanat-Komponente beim erfindungsgemäßen Verfahren eingesetzt werden. Vorzugsweise werden zur Herstellung dieser NCO-Präpolymeren die an sich bekannten Polyhydroxypolyäther oder die an sich bekannten Polyhydroxypolyester eingesetzt. Die OH-Funktionalität diester Polyhydroxylverbindungen wird bei der Herstellung der NCO-Präpolymeren vorzugsweise so gewählt, daß (m + n)-wertige NCO-Präpolymere erhalten werden.

Die in Frage kommenden Hydroxylgruppen aufweisenden Polyester weisen im allgemeinen ein Molekulargewicht von 600 bis 6000 vorzugsweise 1000 bis 4800 auf und stellen Umsetzungsprodukte von Mehrwertigen, vorzugsweise zweiwertigen und ggf. zusatzlich dreiwertigen Alkoholen mit unterschüssigen Mengen an mehrwertigen, vorzugsweise zweiwertigen Carbonsäuren dar. Anstelle der freien Polycarbonsäuren können auch die entsprechenden Polycarbonsäureanhydride oder entsprechende Polycarbonsäureester von niedrigen Alkoholen oder deren Gemische zur Herstellung von Polyester verwendet werden. Die Polycarbonsäuren können aliphatischer, cycloaliphatischer, aromatischer und/oder heterocyclischer Natur sein und ggf., z.B. durch Halogenatome, substituiert und/oder ungesättigt sein. Als Beispiele heirfür seien genannt: Adipinsäure, Phthalsäure, Hexahydrophthalsäureanhydrid, Maleinsäure, Maleinsäureanhydrid.

Als mehrwertige Alkohole kommen z.B. Äthylenglykol, Propylenglykol-(1,2) und -(1,3), Hexandiol-(1,6), Neopentylglykol, 1,4-Bishydroxymethylcyclohexan, 2-Methyl-1,3-propandiol, Glycerin, Trimethylolpropan, ferner Diäthylenglykol, Polyäthylenglykole, Dipropylenglykol, Polypropylenglykole, Dibutylenglykol und Polybutylenglykole in Frage. Die Polyester können anteilig endständige Carboxylgruppen aufweisen. Auch Polyester aus Lactonen, z.B. $\varepsilon$-Caprolacton oder Hydroxycarbonsäuren, wie z.B. $\omega$-Hydroxycapronsäure, sind einsetzbar.

Die in Betracht kommenden Polyhydroxypolyäther weisen im allgemeinen ein Molekulargewicht von 600 bis 6000 vorzugsweise 1000 bis 4800 auf und werden z.B. durch Anlagerung dieser Epoxide, ggf. im Gemisch oder nacheinander, an Startkomponenten mit reaktionsfähigen Wasserstoffatomen, wie Alkanole oder Amine, z.B. Wasser, Äthylenglykol, Propylenglykol-(1,3) oder -(1,2), Trimethylolpropan, 4,4'-Dihydroxydiphenylpropan, Änilin, Ämmoniak, Äthanolamin, Äthylendiamin hergestellt. Vielfach sind solche Polyäther bevorzugt, die überwiegend (bis zu 90 Gew.-%, bezogen auf alle vorhandenen OH-Gruppen im Polyäther) primäre OH-Gruppen aufweisen.

Selbstverständlich können beim erfindungsgemäßen Verfahren als Polyisocyanat-Komponente auch NCO-Präpolymere eingesetst werden, die durch Umsetzung überschüssiger Mengen der beispielhaft genannten Polyisocyanate vorzugsweise Diisocyanate mit anderen Verbindungen mit gegenüber Isocyanatgruppen reaktionsfähigen Gruppen als den beispielhaft genannten hergestellt werden.

Vertreter dieser erfindungsgemäß zu verwendenden Verbindungen sind z.B. in High Polymers, Vol. XVI, "Polyurethanes, Chemistry and Technology", verfaßt von Saunders-Frisch, Interscience Publishers, New York, London, Band I, 1962, Seiten 32—42 und Seiten 44—54 und Band II, 1964, Seiten 5—6 und 198—199 sowie im Kunststoff-Handbuch, Band VII, Vieweg-Höchtlen, Carl-Hanser-Verlag, München, 1966, z.B. auf den Seiten 45—71, beschrieben.

Zur Durchfürung des erfindungsgemäßen Verfahrens wird im allgemeinen das Cycloaminal vorgelegt und bei Temperaturen zwischen 10 und 80°C mit dem Polyisocyanat im Sinne einer Isocyanat-Additionsreaktion umgesetzt. Die Umsetzung kann in Gegenwart oder aber auch in Abwesenheit von inerten Lösungsmittel wie z.B. Aceton, Methyläthylketon, Äthylacetat, Butylacetat, Toluol, Xylol, und/oder Lösungsbenzinen bzw. aus derartigen Lösungsmitteln bestehenden Gemischen durchgeführt werden. Es ist grundsätzlich jedoch auch möglich, die obengenannte Reihenfolge der Zusammenführung der Reaktionspartner umzukehren. Im allgemeinen werden die Mengenverhältnisse der Reaktionspartner so gewählt, daß für jedes Mol Cycloaminal 1 bis 2 Val NCO zur Verfügung stehen.

Bei Verwendung eines Verhältnisses (Mol Cycloaminal) : (Val NCO) von 1:1 entstehen erfindungsgemäße Verfahrensprodukte mit $n = 0$, d.h. die bereits eingans erwähnten latenten Härter für Polyisocyanate. Bei Verwendung eines NCO-Überschusses entstehen die erfindungsgemäßen Verfahrensprodukte mit $n = 1$ oder 2 vorzugsweise 1, die bereits selbst feuchtigkeitshärtende Kunststoffvorläufer darstellen, oder die durch Umsetzung mit weiteren Verbindungen mit gegenüber Isocyanatgruppen reaktionsfähigen Gruppen in erfindungsgemäße Verbindungen mit $n = 0$ überführt werden können, die bezüglich ihrer chemischen Konstitution weitgehend den erfindungsgemäßen Verbindungen entsprechen, die bei Verwendung der entsprechenden NCO-Präpolymeren entstehen. So kann man beispielsweise anstelle von NCO-Präpolymeren als Polyisocyanatkomponente das erfindungsgemäße Verfahren zunächst mit einem dem NCO-Präpolymeren entsprechenden niedermolekularen Diisocyanat unter Verwendung eines Überschusses dieses Diisocyanats durchführen, um anschließend das NCO-gruppenhaltige Umsetzungsprodukt mit der dem NCO-Präpolymeren entsprechenden Verbindung mit gegenüber Isocyanatgruppen reaktionsfähigen Wasserstoffatomen zu modifizieren. Eine weitere Variante bei der Durchfürung des erfindungsgemäßen Verfahrens bestünde auch darin, ein niedermolekulares Polyisocyanat vorzugsweise Diisocyanat der beispielhaft angegebenen Art direkt mit einem Gemisch aus Cycloaminal und Verbindungen mit gegenüber Isocyanatgruppen reaktionsfähigen Wasserstoffatomen umzusetzen. Auch bei der Herstellung von NCO-Gruppenfreien erfindungsgemäßen Verbindungen kann es bei der Durchführung des erfindungsgemäßen Verfahrens zweckmäßig sein, einen geringen NCO-Überschuß zur Anwendung zu bringen, um ggf. vorhandene Feuchtigkeitsspuren in dem Cycloaminal zu kompensieren. Es ist bei der Durchführung des erfindungsgemäßen Verfahrens auch möglich, unter Verwendung eines hohen Überschusses an Cycloaminal zu arbeiten (höher als dem obengenannten Verhältnis von 1:1 entsprechend), um anschließend das nicht umgesetzte Cycloaminal beispielsweise durch Dünnschichtdestillation zu entfernen, oder auch um das überschüssige Cycloaminal im Reaktionsgemisch zu belassen, da auch das nicht umgesetzte Cycloaminal die Rolle eines niedermolekularen teilblockierten Vernetzers spielen kann und bei bestimmten Anwendungsgebieten für die erfindungsgemäßen Verbindungen nicht stört.

Die besonders bevorzugten erfingungsgemäßen Verbindungen sind diejenigen, die keine freien NCO-Gruppen aufweisen und die bei Raumtemperatur in Abwesenheit von Feuchtigkeit gegenüber Isocyanatgruppen weitgehend inert sind, unter dem Einfluß von Feuchtigkeit und/oder Hitze jedoch gegenüber Isocyanatgruppen reaktionsfähig sind. Bei einer Aktivierung der erfindungsgemäßen Verfahrensprodukte durch einfluß von Feuchtigkeit tritt, wie bereits dargelegt, eine hydrolytische Spaltung des Aminal-Rings unter Freisetzung von gegenüber Isocyanatgruppen hochreaktiven sekundären Aminogruppen ein. Bei der Aktivierung der erfindungsgemäßen Verbindungen durch ausschließliche Einwirkung von Hitze ohne Feuchtigkeitszutritt kommen sehr wahrscheinlich andersartige Vernetzungsreaktionen in Betracht, deren Mechanismus noch nicht völlig geklärt ist. Es steht jedoch fest, daß Gemische aus NCO-Gruppen aufweisenden Kunststoffvorläufern und erfindungsgemäßen Verbindungen auch in Abwesenheit von Feuchtigkeit unter ausschließlicher Hitzeeinwirkung, beispielsweise durch Erhitzen unter Luft- und Feuchtigkeitsabschluß auf 100 bis 160°C zu hochmolekularen Kunststoffen umgesetzt werden können.

Die bevorzugten erfindungsgemäßen, NCO-Gruppen-freien Verbindungen werden vorzugsweise unter ausschließlicher Verwendung der beispielhaft genannten einfachen organischen Diisocyanate hergestellt, während bei der Herstellung von erfindungsgemäßen NCO-Gruppen-haltigen Verbindungen vorzugsweise die beispielhaft genannten höhermolekularen Hydroxylgruppen aufweisenden Verbindungen über die NCO-Präpolymeren mit eingebaut werden, so daß die Umsetzungsprodukte selbst echte, oligomere, durch den Einfluß von Feuchtigkeit und/oder Hitze zu hochmolekularen Kunststoffen ausreagierende Kunststoffvorläufer darstellen. Die NCO-Gruppen-freien erfindungsgemäßen Verbindungen eignen sich nicht nur als latente Vernetzer für NCO-Gruppen aufweisende Kunststoffvorläufer (beispielsweise den oben beispielhaft genannten NCO-Präpolymeren) sondern können auch als latente Kettenverlängerungsmittel bzw. Vernetzer Reaktivsystemen auf Basis von organischen Polyisocyanaten der beispielhaft genannten Art und von organischen Verbindungen mit gegenüber Isocyanatgruppen reaktionsfähigen Wasserstoffatomen der beispielhaft genannten Art zugesetzt werden, wodurch überraschenderweise gleichzeitig eine Erhöhung der Topfzeit und eine Erniedrigung der Trocknungszeit der Systeme erzielbar ist. In solchen Systemen liegt im allgemeinen ein 1,05—1,4-facher molarer NCO-Überschuß bezogen auf die aktiven Wasserstoffatome der Komponente mit gegenüber Isocyanatgruppen reaktionsfähigen Gruppen und bezogen auf diesen NCO-Überschuß, eine mindestens 0,8-fach äquivalente Menge der erfindungsgemäßen Verbindung vor.

Die hier beschriebenen Reaktivesysteme können inerte Zusätze enthalten, wie z.B. Lösungsmittel, Weichmacher, Flammschutzmittel, Füllstoffe, Entlüftungsmittel, Fließhilfsmittel oder Pigmente, um sie den jeweiligen Forderungen der Praxis anzupassen. Die beschriebenen Reaktivsysteme eignen sich zur Herstellung von Lacken, Beschichtungs- oder Vergußmassen für eine Vielzahl von Substraten wie z.B. Holz, Papier, Textilien, Leder, Glas, Stein, Beton oder Metall. Besonders eignen sich die beschriebenen Reaktivsysteme für einen Einsatz auf dem Bausektor und für Anwendungen in der Automobilindustrie. Je nach Verwendungart können die Reaktivsysteme durch Streichen, Spritzen, Spachteln, Gießen oder Rakeln aufgetragen werden.

Beispiel 1
N-Methyl-hexahydropyrimidin

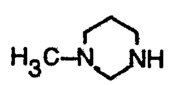

Zu einer Suspension von 198 g Paraformaldehyd in 700 ml Cyclohexan laßt man bei 60°C langsam 528 g N-Methyl-1,3-propandiamin zutropfen. Durch Kochen unter Rückfluß am Wasserabscheider wird das entstehende Wasser entfernt. Nach Abfiltrieren vom Unlöslichen wird das Filtrat über eine 30-cm-Vigreux-Kolonne destilliert. Man erhält 455 g N-Methyl-hexahydropyrimidin, das bei Normaldruck zwischen 130 und 138°C siedet.

Beispiel 2
1-Methyl-2-(1-methyläthyl)-hexahydropyrimidin

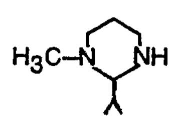

Zu einer Mischung aus 650 ml Isobutyraldehyd und 400 ml Cyclohexan läßt man unter Eiskühlung langsam 530 g N-Methyl-1,3-diaminpropan tropfen. Nach Abklingen der exothermen Reaktion läßt man die Reaktionsmischung unter Rückfluß am Wasserabscheider kochen bis sich etwa 108 ml Wasser abgretrennt haben. Cyclohexan und Isobutyraldehyd werden unter vermindertem Druck abdestilliert. Der Rückstand liefert nach fraktionierter Destillation ($Kp_{15}$: 68 bis 75°C) 680 g 1-Methyl-2-isopropyl-hexahydropyrimidin.

Beispiel 3
N-Cyclohexyl-hexahydropyrimidin

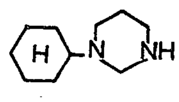

Zu einer Suspension von 66 g Paraformaldehyd in 200 ml Cyclohexan läßt man bei 60°C langsam 312 g N-Cyclohexyl-1,3-propandiamin zutropfen. Anschliegend entfernt man das entstehende Wasser durch Kochen unter Rückfluß am Wasserabscheider. Man filtriert vom Unlöslichen ab und unterwirft das Filtrat einer fraktionierten Destillation. Man erhalt 248 g N-Cyclohexyl-hexahydropyrimidin, das bei 0,3 mbar zwischen 87 und 91°C siedet.

Beispiel 4
N-(6-Isocyanato-1,6-hexandiyl)-3-methyl-hexahydropyrimidin-1-carboxamid

Zu 336 g Hexamethylendiisocyanat läßt man bei Raumtemperatur langsam 100 g N-Methylhexahydropyrimidin zutropfen. Nach beendeter Zugabe läßt man noch 3 Stunden weiterrühren. Anschließend gibt man 250 ml Cyclohexan zu und läßt bei Raumtemperatur 1 Stunde rühren. Man trennt die Cyclohexanphase ab und wiederholt diese Reinigungsoperation dreimal mit je 250 ml Cyclohexan. Nach dem Abtrennen vom Lösungsmittel wird das Produkt noch durch Dünnschichtdestillation bei 100°C und 15 mbar vom restlichen Cyclohexan befreit. Das Produkt enthält weniger als 0,4 % freies Diisocyanat und besteht bevorzugt aus folgendem Monoisocyanat:

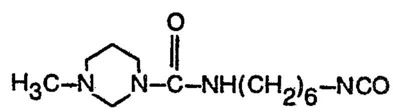

Beispiel 5
N - (5 - Isocyanato - 1,3,3 - trimethyl - 1 - cyclohexylmethyl) - 3 - methyl - 2 - (1 - methyläthyl) - hexahydropyrimidin-1-carboxamid

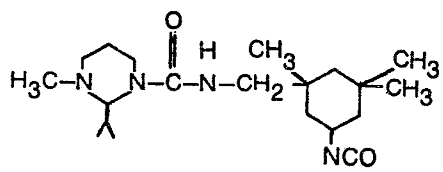

Zu 888 g Isophrondiisocyanat läßt man bei Raumtemperatur langsam 142 g N-Methyl-2-isopropyl-hexahydropyrimidin zutropfen. Anschließend rührt man noch 6 Stdn. nach und entfernt die Hauptmenge des überschüssigen Diisocyanats durch Dünnschichtdestillation (160°/0,1 mbar). Der Rückstand wird in Toluol gelöst und durch Zugabe von Cyclohexan ausgefällt, anschließend abgesaugt, mit Cyclohexan gewaschen und dann getrocknet. Man erhält rund 300 g eines Produktes, das bevorzugt aus dem 1 : 1-Additionsprodukt aus Isophorondiisocyanat und dem Hexahydropyridimin-derivat besteht (Fp.: 180—192°).

Beispiel 6
Modifiziertes Polyol

An 627 g eines Polyesters, der aus Phthalsäure, Trimethylolpropan, $\alpha$-Äthylhexandicarbonsäure und 2-Äthylhexandiol-(1,3) erhalten wird und einen OH-Gehalt von 8,1 % hat, addiert man unter Zusatz von 0,2 g Dibutylzinndilaurat 108 g des Hexahydropyrimidinisocyanats, das nach Beispiel 4 hergestellt wurde. Die Reaktion wird bei Temperaturen um 40°C durchgeführt. Man erhält so ein modifiziertes Polyesterpolyol, das nebeneinander OH— und Hexahydropyrimidinendgruppen enthält. Das Produkt hat eine OH-Zahl von 256 und eine Säurezahl von 11,6, wobei aus praktischen Gründen in der OH-Zahl auch die durch Hydrolyse entstehenden NH-Funktionen erfaßt werden.

Beispiel 7
Präpolymer mit Isocyanat- und Hexahydropyrimidinendgruppen

Zu 1150 g eines Isocyanatpräpolymers mit einem NCO-Gehalt von 3,8 %, das man durch Umsetzung von Isophorondiisocyanat mit einem auf Propylenglykol gestarteten Polyäther auf Propylenoxidbasis mit der OH-Zahl 56 erhält, gibt man bei 30 bis 40°C langsam 72 g N-Methyl-2-isopropyl-hexahydropyrimidin zu und läßt bei Raumtemperatur 8 Stunden nachrühren.

Man erhält ein Vorpolymerisat, das durch Feuchtigkeit und/oder Wärme ausgehärtet werden kann.

Beispiel 8

Präpolymer mit Hexahydropyrimidin-Endgruppen

Zu 1100 g eines Isocyanatpräpolymers mit einem NCO-Gehalt von 3,8 %, das durch Umsetzung von Toluylen-2,4-diisocyanat mit einem auf Propylenglykol gestarteten Polyäther auf Propylenoxidbasis von der OH-Zahl 56 erhalten wird, gibt man bei Raumtemperatur langsam 150 g N-Methyl-2-isopropylhexahydropyrimidin zu und läßt bei gleicher Temperatur nachrühren bis die Isocyanatbande im IR-Spektrum verschwunden ist.

Das Produkt hat eine Viskosität von 120 000 mPa·s (25°C) und ein durch Titration bestimmtes NH-Äquivalentgewicht von 1280.

Beispiel 9

N,N'-(1,6-hexandiyl)-bis-[3-(2-cyanäthyl)-2-(1-methyläthyl)-1-imidazolidin-carboxamid]

Zu einer Mischung aus 144 g Isobutyraldehyd in 400 ml Cyclohexan läßt man langsam 226 g N-(2-Cyanäthyl)-äthylendiamin zutropfen. Durch Kühlung hält man die Temperatur unter 30°. Nach beendeter Zugabe läßt man unter Rückfluß am Wasserabscheider kochen bis sich rund 30 ml Wasser abgetrennt haben. Anschließend wird das Lösungsmittel abdestilliert. Zum rohen Imidazolidin gibt man unter Kühlung bei Temperaturen zwischen 30 und 40°C langsam 168 g Hexamethylendiisocyanat. Man erhält so ein viskoses hellgelbes produkt, dem bevorzugt die folgende Formel

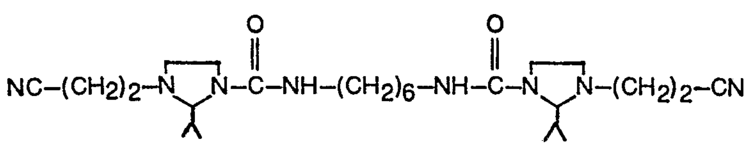

zukommt.

Biespiel 10

N,N'-(1,6-hexandiyl)-bis-[3-methyl-2-(1-methyläthyl)-1-hexahydropyrimidin-carboxamid]

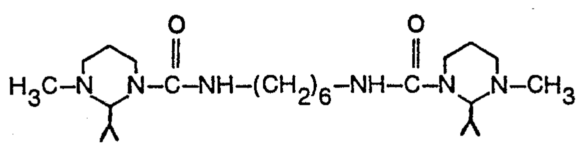

142 g N-Methyl-2-isopropyl-hexahydropyrimidin

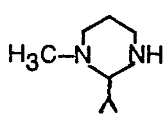

in 150 ml Essigsäureäthylester versetzt man bei Temperaturen zwischen 25 und 35°C langsam mit einer Mischung aus 84 g Hexamethylendiisocyanat und 100 g Essigester. Nach beendeter Reaktion läßt man noch 8 Stdn. bei Raumptemperatur nachrühren und entfernt anschließend den Ester bei 100°C und 25 mbar am Rotationsverdampfer.

Beispiel 11

N,N'-(1,6-Hexandiyl)-bis-(3-methyl-1-hexahydropyrimidincarboxamid)

Zu einer Mischung aus 224 g N-Methyl-hexahydropyrimidin und 150 ml Essigsäureäthylester läßt man langsam unter Kühlung bei Temperaturen zwischen 15 und 20°C 168 g Hexamethylendiisocyanat in 150 ml Essigsäureäthylester zutropfen. Nach beendeter Zugabe läßt man bei Raumtemperatur weiterrühren bis die Isocyanatbande im IR-Spektrum verschwunden ist (ca. 2 Stdn.). Am Dünnschichtverdampfer entfernt man bei 110°C und Wasserstrahlpumpenvakuum das Lösungsmittel. Das viskose Produkt besteht bevorzugt aus dem folgenden Bishexahydropyrimidinderivat.

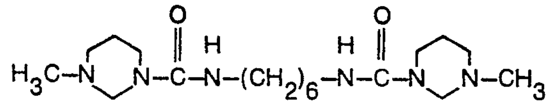

Für die nachfolgenden Beispiele gelten fogende Abkürzungen:

Produkt 1 = Polyacrylatharz auf Basis Styrol, Butylacrylat, Hydroxy-propyl-acrylat mit 4,8 Gew.-% OH.
Produkt 2 = N,N' - (1,6 - Hexandiyl) - bis - [3 - methyl - 2 (1 - methyläthyl) - 1 - hexahydropyrimidin - carboxamid]
Produkt 3 = N,N'-(1,6-hexandiyl)-bis-(3-methyl-1-hexahydropyrimidin-carboxamid)
Produkt 4 = Handelsübliches Entlüftungsmittel auf Polyacrylatharz-Basis
Produkt 5 = Handelsübliches Verlaufmittel auf Silikonöl-Basis
Produkt 6 = Biuretgruppenhaltiges Polyisocyanat auf Basis Hexamethylendiisocyanat mit 23,5 Gew.-% NCO.

Beispiel 12

100,0 g eines NCO-Prepolymeren hergestellt aus einem Polyolgemisch bestehend aus 1 Mol linearem Polypropylenglykol des Molekulargewichtes 2000 und 0,4 Mol propoxyliertem Trimethylpropan des Molekulargewichtes 2000 und uberschüssigem 3,3,5-Trimethyl-5-isocyanatomethylcyclohexylisocyanat (NCO-Gehalt = 4,0 %; Viskosität bei 20°C = ca. 7500 mPa·s) und 27 g des Produkts 2 werden nach Zugabe von 1,0 g Ölsäure Vermischt.

Dickschichtige Ueberzüge bilden bei Raumtemperatur und einer relativen Luftfeuchtigkeit von 50 % innerhalb von 2 Stunden eine Haut und härten zu elastischen Belägen aus. Die Lagerfähigkeit o.g. Bindemittelsystems liegt bei 50°C über 10 Tagen.

Beispiel 13

Herstellung einer feuchtigkeitshärtbaren Dichtstoffmasse

100,0 g des NCO-Prepolymeren aus Beispiel 12
150,0 g Alkylsulfonsäureester ($C_{15}H_{31}—SO_3—C_6H_5$) (Weichmacher)
75,0 g Kreidepulver
18,0 g hochdisperse Kieselsäure
5,0 g Zeolith-Pulver (Molekularsieb)
5,0 g Titandioxid
0,2 g Ruß
10,0 g Xylol
25,0 g Produkt 2

Die Dichtstoffmasse bildet bei Reaumtemperatur und 50 % relativer Luftfeuchtigkeit nach ca. 4 Stunden eine Haut und härtet 1—2 mm pro Tag durch. Shore A-Härte des gehärteten Materials = ca. 20. Die Lagerfähigkeit der angegebenen Mischung liegt bei 20°C über 3 Monate.

Beispiel 14 (Vergleich)

Die Zusammensetzung der Komponente A ist wie folgt:

| | | |
|---|---|---|
| Produkt 1, | 80 %ig in EGA+ | 125,0 Gew.-Tle. |
| Produkt 4, | 10 %ig in EGA+ | 0,7 Gew.-Tle. |
| Produkt 5, | 10 %ig in Xylol | 1,4 Gew.-Tle. |
| Zinkoctoat, | 10 %ig in Xylol | 2,9 Gew.-Tle. |
| EGA+ | | 37,2 Gew.-Tle. |
| Xylol | | 37,1 Gew.-Tle. |
| | | 204,3 Gew.-Tle. |
| Komponente B<br>EGA/Xylol (1:1) | | 13,7 Gew.-Tle. |
| Produkt 6, | 100 %ig | 44,1 Gew.-Tle. |
| | | 57,8 Gew.-Tle. |

204,3 g der Komponente A werden mit 57,8 g der Komponente B innig vermischt und mit der Fließbecherpistole in einer Schichtdicke von ca. 80 $\mu$m Naßfilm aufgespritzt.

Folgende physikalischen Trocknungswerte und Filmeigenschaften wurden erhalten:

Trocknung nach DIN 53150
Trocknungsstufe 1: ca. 7 Stdn.
+) = Äthylenglykol-monoäthyläther-acetat

Schwingversuche mit dem Pendelgerät DIN 53 157

| | | |
|---|---|---|
| 1.) nach 1 Tag Lagerung bei 23°C: | ca. | 40 Sek. |
| 2.) nach 30 Minuten bei 80°C: | ca. | 125 Sek. |
| 3.) nach 12 Stunden bei 70°C: | ca. | 185 Sek. |

Topfzeit im geschlossenen Gefäß bei 20°C ca. 14 Stunden.

Beispiel 15

Di Zusammensetzung der Komponente A ist wie folgt:

| | | | |
|---|---|---|---|
| Produkt 1, | 80 %ig in EGA | 100,0 | Gew.-Tle. |
| Produkt 3, | 100 %ig | 20,0 | „ |
| Produkt 4, | 10 %ig in EGA | 0,8 | „ |
| Produkt 5, | 10 %ig in Xylol | 1,5 | „ |
| Zinkoctoat, | 10 %ig in Xylol | 3,1 | „ |
| EGA | | 38,3 | „ |
| Xylol | | 38,3 | „ |
| | | 202,0 | Gew.-Tle. |

Komponente B

| | | | |
|---|---|---|---|
| Produkt 6, | 100 %ig | 54,5 | Gew.-Tle. |
| EGA/Xylol (1:1) | | 17,1 | Gew.-Tle. |
| | | 71,6 | Gew.-Tle. |

202,0 g der Komponente A werden mit 71,6 g der Komponente B innig vermischt und mit der Fließbecherpistole in einer Schichtdicke von ca. 80 $\mu$m Naßfilm aufgespritzt.

Folgende physikalischen Trocknungswerte und Filmeigenschaften wurden ermittelt:

Trocknung nach DIN 53150
Trocknungstuffe 1: ca. 4 Stdn.

Schwingungsversuche mit dem Pendelgerat DIN 53157

| | | |
|---|---|---|
| 1.) nach 1 Tag Lagerung bei 23°C: | ca. | 67 Sek. |
| 2.) nach 30 Min. bei 80°C: | ca. | 143 Sek. |
| 3.) nach 12 Stdn. bei 70°C: | ca. | 186 Sek. |
| Topfzeit bis zur Gelierung im geschlossenen Gefäß bei 20°C | ca. | 25 Stdn. |

**Patentansprüche**

1. Verbindungen der Formel

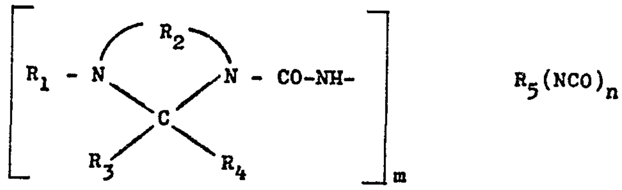

in welcher

m für eine ganze Zahl von 1 bis 3 und

n 0, 1 oder 2 steht, wobei die Summe m + n 2 oder 3 beträgt,

$R_1$ einen gegebenenfalls Cyano-substituierten aliphatischen Kohlenwasserstoffrest mit 1 bis 6 Kohlenstoffatomen, einen cycloaliphatischen Kohlenwasserstoffrest mit 5—10 Kohlenstoffatomen oder einen araliphatischen Kohlenwasserstoffrest mit 7—10 Kohlenstoffatomen bedeutet,

$R_2$ für einen zweiwertigen aliphatischen Kohlenwasserstoffrest mit 2 bis 6 Kohlenstoffatomen steht, wobei zwischen den beiden Stickstoffatomen mindestens zwei Kohlenstoffatome angeordnet sind,

$R_3$ und $R_4$ für gleiche oder verschiedene Reste stehen und Wasserstoff, aliphatische Kohlenwasserstoffreste mit 1—18 Kohlenstoffatomen, cycloaliphatische Kohlenwasserstoffreste mit 5—10 Kohlenstoffatomen oder aromatische Kohlenwasserstoffreste mit 6—10 Kohlenstoffatomen bedeuten oder wobei die beiden Reste $R_3$ und $R_4$ zusammen mit dem Kohlenstoffatom des heterocyclischen Rings auch einen 5- oder 6-gliedrigen cycloaliphatischen Ring bilden können,

$R_5$ für einen (m + n)-wertigen Rest steht, wie er durch Entfernung von m + n Isocyanatgruppen aus einem (m + n)-wertigen Polyisocyanat erhalten wird.

2. Verfahren zur Herstellung von Verbindungen gemäß Anspruch 1, dadurch gekennzeichnet daß man cyclische Aminale der Formel

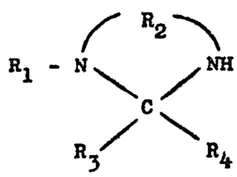

mit Polyisocyanaten der Formel

$$R_5\,(NCO)_{m+n}$$

zur Reaktion bringt, wobei

$R_1$, $R_2$, $R_3$, $R_4$, $R_5$, m und n die in Anspruch 1 genannte Bedeutung haben.

3. Verwendung der Isocyanatgruppen-freien Verbindungen gemäß Anspruch 1 als durch den Einfluß von Feuchtigkeit und/oder Hitze aktivierbare Härter für freie Isocyanatgruppen oder mit an sich bekannten Blockierungsmitteln für Isocyanatgruppen blockierte Isocyanatgruppen aufweisende Kunststoffvorläufer.

4. Verwendung der Isocyanatgruppen aufweisenden Verbindungen gemäß Anspruch 1 als durch Einfluß von Feuchtigkeit und/oder Hitze zu hochmolekularen Kunststoffen ausreagierende Kunststoffvorläufer.

**Revendications**

1. Composés de formule:

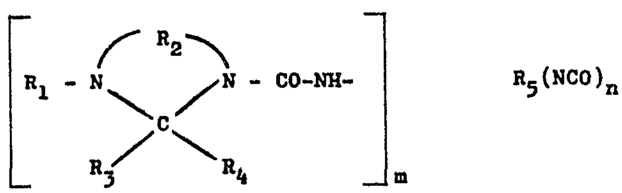

dans laquelle

m représente un nombre entier de 1 à 3 et

n 0, 1 ou 2, la somme m + n faisant 2 ou 3,

$R_1$ représente un radical hydrocarboné aliphatique éventuellement cyano-substitué ayant 1 à 6 atomes de carbone, un radical hydrocarboné cycloaliphatique ayant 5 à 10 atomes de carbone ou un radical hydrocarboné araliphatique ayant 7 à 10 atomes de carbone,

$R_2$ représente un radical hydrocarboné aliphatique bivalent ayant 2 à 6 atomes de carbone, où, entre les deux atomes d'azote, sont agencés au moins deux atomes de carbone,

$R_3$ et $R_4$ sont des radicaux identiques ou différents et ils représentent de l'hydrogène, des radicaux hydrocarbonés aliphatiques ayant 1 à 18 atomes de carbone, des radicaux hydrocarbonés cycloaliphatiques ayant 5 à 10 atomes de carbone ou des radicaux hydrocarbonés aromatiques ayant 6 à 10 atomes de carbone, ou bien les deux radicaux $R_3$ et $R_4$ pouvant former conjointement avec l'atome de carbone du noyau hétérocyclique également un noyau cycloaliphatique à 5 ou 6 chaînons,

$R_5$ représente un radical de valence (m + n), tel qu'on l'obtient par élimination de m + n groupes isocyanate à partir d'un polyisocyanate de valence (m + n).

2. Procédé de préparation de composés selon la revendication 1, caractérisé en ce qu'on fait réagir des aminals cycliques de formule:

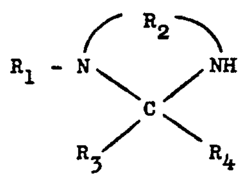

avec des polyisocyanates de formule

$$R_5\,(NCO)_{m+n},$$

$R_1$, $R_2$, $R_3$, $R_4$, $R_5$, m et n ayant les significations indiquées à la revendication 1.

3. Utilisation des composés exempts de groupes isocyanate selon la revendication 1 comme durcissants activables sous l'influence de l'humidité et/ou de la chaleur, pour précurseurs de matières plastiques présentant des groupes isocyanate libres ou présentant des groupes isocyanate bloqués avec des agents de blocage, connus en eux-mêmes, pour groupes isocyanate.

4. Utilisation des composés présentant des groupes isocyanate selon la revendication 1 comme précurseurs de matières plastiques réagissant sous l'influence de l'humidité et/ou de la chaleur en donnant des matières plastiques à poids moléculaire élevé.

**Claims**

1. Compounds of the formula:

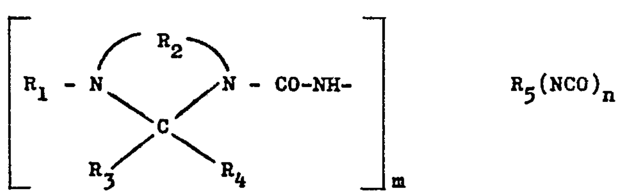

wherein

m represents an integer of from 1 to 3 and

n represents 0, 1 or 2 and the sum of m + n is 2 or 3;

$R_1$ represents an aliphatic hydrocarbon group which is optionally cyano-substituted and which has from 1 to 6 carbon atoms, a cycloaliphatic hydrocarbon group having from 5 to 10 carbon atoms or an araliphatic hydrocarbon group having from 7 to 10 carbon atoms;

$R_2$ represents a divalent aliphatic hydrocarbon group having from 2 to 6 carbon atoms, at least two carbon atoms being arranged between the two nitrogen atoms;

$R_3$ and $R_4$ represent identical or different groups and denote hydrogen, aliphatic hydrocarbon groups having from 1 to 18 carbon atoms, cycloaliphatic hydrocarbon groups having from 5 to 10 carbon atoms or aromatic hydrocarbon groups having from 6 to 10 carbon atoms or the two groups $R_3$ and $R_4$ together with the carbon atom of the heterocyclic ring may form a 5-membered or 6-membered cycloaliphatic ring;

$R_5$ represents an (m + n)-valent group such as that which is obtained by the removal of m + n isocyanate groups from an (m + n)-valent polyisocyanate.

2. A process for the preparation of compounds according to claim 1, characterised in that cyclic aminals of the formula:

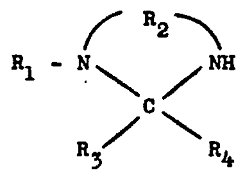

are reacted with polyisocyanates of the formula:

$$R_5\,(NCO)_{m+n}$$

wherein

$R_1$, $R_2$, $R_3$, $R_4$, $R_5$, m and n have the meaning given in claim 1.

3. The use of the compounds according to claim 1 which are free from isocyanate groups as hardeners which can be activated by moisture and/or heat for synthetic resin precursors which contain free isocyanate groups or isocyanate groups which are masked with known blocking agents for isocyanate groups.

4. The use of the compounds according to claim 1 which contain isocyanate groups as synthetic resin precursors which react under the influence of moisture and/or heat to form high molecular weight synthetic resins.